(19) Europäisches Patentamt / European Patent Office / Office européen des brevets

(11) **EP 2 768 389 B1**

(12) **FASCICULE DE BREVET EUROPEEN**

(45) Date de publication et mention de la délivrance du brevet:
**13.03.2019 Bulletin 2019/11**

(21) Numéro de dépôt: **12787780.1**

(22) Date de dépôt: **19.10.2012**

(51) Int Cl.:
**A61B 5/11** *(2006.01)*

(86) Numéro de dépôt international:
**PCT/FR2012/052394**

(87) Numéro de publication internationale:
**WO 2013/057447 (25.04.2013 Gazette 2013/17)**

(54) **PROCÉDÉ DE DÉTECTION D'ACTIVITÉ À CAPTEUR DE MOUVEMENTS, DISPOSITIF ET PROGRAMME D'ORDINATEUR CORRESPONDANTS**

VERFAHREN ZUM NACHWEIS EINER AKTIVITÄT MIT EINEM BEWEGUNGSSENSOR SOWIE ENTSPRECHENDE VORRICHTUNG UND COMPUTERPROGRAMM

METHOD OF DETECTING ACTIVITY WITH A MOTION SENSOR, AND CORRESPONDING DEVICE AND COMPUTER PROGRAM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.10.2011 FR 1159595**

(43) Date de publication de la demande:
**27.08.2014 Bulletin 2014/35**

(73) Titulaires:
• **Commissariat à l'Énergie Atomique et aux Énergies Alternatives**
 **75015 Paris (FR)**
• **Movea**
 **38000 Grenoble (FR)**

(72) Inventeur: **JALLON, Pierre**
 **F-38700 Corenc (FR)**

(74) Mandataire: **Bonnet, Michel**
 **Cabinet Bonnet**
 **93, rue Réaumur - Boîte 10**
 **75002 Paris (FR)**

(56) Documents cités:
**WO-A1-2010/122174     US-A1- 2010 010 383**

• **WANG L ET AL: "Recent developments in human motion analysis", PATTERN RECOGNITION, ELSEVIER, GB, vol. 36, no. 3, 1 mars 2003 (2003-03-01), pages 585-601, XP004393092, ISSN: 0031-3203, DOI: 10.1016/S0031-3203(02)00100-0**
• **BILMES J A: "A Gentle Tutorial of the EM Algorithm and its Application to Parameter Estimation for Gaussian Mixture and Hidden Markov Models", INTERNET CITATION, 1 avril 1998 (1998-04-01), pages 1-15, XP002594206, Extrait de l'Internet: URL:ftp://ftp.icsi.berkeley.edu/pub/techreports/1997/tr-97-021.pdf [extrait le 2009-07-27]**
• **RABINER L ET AL: "An introduction to hidden Markov models", IEEE ASSP MAGAZINE, IEEE, US, vol. 3, no. 1, 1 janvier 1986 (1986-01-01) , pages 4-16, XP011353445, ISSN: 0740-7467, DOI: 10.1109/MASSP.1986.1165342**

Remarques:
Le dossier contient des informations techniques présentées postérieurement au dépôt de la demande et ne figurant pas dans le présent fascicule.

**Description**

La présente invention concerne un procédé de détection d'activité à capteur de mouvements. Elle concerne aussi un dispositif de détection et un programme d'ordinateur correspondants.

Plus précisément, elle porte sur un procédé de détection de l'activité d'un système physique porteur d'un capteur de mouvements, comportant les étapes suivantes :

- extraire une séquence de mesures fournies par le capteur de mouvements,
- en déduire une séquence d'observation calculée à partir de la séquence de mesures,
- à l'aide d'un modèle statistique de composantes de la séquence d'observation au vu d'une pluralité d'états prédéterminés possibles dans lesquels peut se trouver le système physique, détermination de l'activité du système physique sous la forme d'une séquence d'états correspondant à la séquence d'observation.

Un procédé de ce type est par exemple décrit dans l'article de P. Jallon, intitulé « A graph based algorithm for postures estimation based on accelerometers data », publié dans Proceedings of 2010 annual international conférence of the IEEE EMBC, pages 2778-2781. Dans cet article, le capteur est un accéléromètre à trois axes de mesure porté sur le buste (taille ou torse) d'une personne observée : l'accéléromètre fournit ainsi une séquence de mesures constituées de projections sur ses trois axes d'une somme de l'accélération due au champ de gravitation terrestre et d'une accélération propre du capteur. Ces mesures vectorielles à trois composantes ne sont pas directement interprétées pour déterminer l'activité de la personne observée mais traitées au préalable pour séparer les informations liées à l'accélération gravitationnelle de celles relatives à l'accélération propre du capteur. Ainsi, trois composantes basse fréquence d'une séquence d'observation sont calculées par filtrage passe-bas de chaque mesure vectorielle et trois composantes haute fréquence de la séquence d'observation sont calculées en soustrayant les trois composantes basse fréquence de la mesure vectorielle. La séquence d'observation résultante est une séquence de valeurs vectorielles à six composantes. Les trois premières sont interprétées comme significatives de projections de l'accélération gravitationnelle sur les axes de mesure du capteur, de sorte qu'elles indiquent la posture (assise/debout ou couchée) de la personne, tandis que les trois suivantes sont interprétées comme significatives de l'accélération propre du capteur, de sorte qu'elles indiquent le degré d'activité (nul, faible, important, ...) de la personne.

Par ailleurs, un modèle statistique des composantes de la séquence d'observation est défini en fonction d'une pluralité d'états prédéterminés possibles dans lesquels peut se trouver la personne : position assise, transition assis/debout, position debout statique, marche, transition debout/assis. Compte tenu des contraintes de transitions d'un état à l'autre, ces états sont modélisés en tant qu'états cachés d'un modèle statistique de Markov et une méthode d'analyse classique par inférence bayésienne est appliquée pour déterminer l'activité de la personne sous la forme d'une séquence d'états correspondant à la séquence d'observation.

Bien sûr, cette méthode d'analyse nécessite un apprentissage préalable sur des séquences connues d'états pour définir les paramètres du modèle statistique, en particulier ceux des lois de probabilité de l'observation liées aux différents états cachés du modèle.

Cette méthode d'analyse et son apprentissage nécessitent également un étalonnage du capteur, notamment pour connaître son orientation dans une posture prédéterminée de référence telle que la posture debout par exemple. Ensuite, le bon fonctionnement du procédé impose que le capteur soit toujours porté de la même manière par la personne observée, une certaine plage de variations autour d'un positionnement optimal étant toutefois tolérée. Il suppose en outre que les valeurs apprises sous étalonnage sont invariantes d'une personne à l'autre ou d'une expérience à l'autre, ce qui est une approximation à la fois grossière et contraignante.

Un autre procédé de ce type est décrit dans la demande de brevet internationale publiée sous le numéro WO 2010/122174 A1. Une même séquence de mesures est obtenue d'un accéléromètre et une séquence d'observation dont les valeurs sont vectorielles et à quatre composantes en est déduite. Les trois premières composantes correspondent à celles obtenues dans l'article précité, tandis que la quatrième est la norme de la sortie d'un filtre passe haut appliqué à la séquence de mesures. Comme précédemment, l'activité de la personne observée est déterminée sous la forme d'une séquence d'états correspondant à la séquence d'observation à l'aide d'un modèle statistique prédéfini. Comme précédemment également, la méthode nécessite un apprentissage, un étalonnage et présente les mêmes inconvénients.

Il peut ainsi être souhaité de prévoir un procédé de détection d'activité qui permette de s'affranchir d'au moins une partie des problèmes et contraintes précités.

L'invention a donc pour objet un procédé de détection de l'activité d'un système physique porteur d'un capteur de mouvements, comportant les étapes suivantes :

- extraire une séquence de mesures fournies par le capteur de mouvements,
- en déduire une séquence d'observation calculée à partir de la séquence de mesures,
- à l'aide d'un modèle statistique de composantes de la séquence d'observation au vu d'une pluralité d'états prédéterminés possibles dans lesquels peut se trouver le système physique, détermination de l'activité du système physique sous la forme d'une séquence d'états correspondant à la séquence d'observation,

dans lequel au moins une composante de la séquence d'observation est calculée à partir d'une valeur de référence d'un vecteur représentatif d'un axe de référence, le procédé comportant les étapes suivantes de calibrage :

- calcul d'une valeur de vraisemblance de la séquence d'observation au vu dudit modèle statistique,
- comparaison de cette valeur de vraisemblance à un seuil prédéterminé,
- en fonction de cette comparaison, déclenchement d'une estimation d'une nouvelle valeur de référence du vecteur représentatif de l'axe de référence du capteur de mouvements.

Ainsi, en rendant au moins une composante de la séquence d'observation explicitement dépendante d'une valeur vectorielle de référence indiquant une direction privilégiée et en prévoyant des moyens permettant la remise en cause de cette valeur de référence sur la base d'un test de vraisemblance exécutable pendant l'observation du système, on rend le procédé capable de procéder à tout moment à un calibrage. On rend ainsi ce procédé moins sensible à la façon dont le capteur est porté par le système physique et plus robuste à tout changement de système observé, d'expérience ou de disposition du capteur de mouvements pendant une observation.

De façon optionnelle, l'axe de référence est estimable, via son vecteur représentatif, à l'aide des mesures fournies par le capteur de mouvements. Ainsi, le calibrage peut se faire de façon automatique et autonome grâce au capteur de mouvements.

De façon optionnelle également, un procédé selon l'invention peut en outre comporter une étape d'invalidation d'au moins une partie de la séquence d'états correspondant à la séquence d'observation, en fonction de la valeur de vraisemblance.

Cela permet de ne pas tenir compte de la détermination d'une activité, au moins en partie, lorsque cette dernière est obtenue à partir d'observations pour lesquelles la valeur de référence n'est pas représentative de l'axe de référence.

De façon optionnelle également, la séquence d'observation est déduite de la séquence de mesures de la façon suivante :

- calcul d'une première composante, à partir d'une éventuelle décimation de la séquence de mesures, d'un filtrage passe bas avec une fréquence de coupure prédéterminée, par exemple 0,125 Hz, de chaque composante de la séquence de mesures éventuellement décimée, puis d'une projection des composantes résultantes sur la valeur de référence,
- calcul d'au moins une seconde composante, à partir d'une éventuelle décimation de la séquence de mesures, puis d'un filtrage passe bande avec des fréquences de coupure prédéterminées, par exemple une fréquence de coupure basse de 0,125 Hz et une fréquence de coupure haute de 0,75 Hz, de chaque composante de la séquence de mesures éventuellement décimée.

De façon optionnelle également, le modèle statistique est un unique modèle de Markov à états cachés dont chaque état caché correspond à l'un des états prédéterminés possibles dans lesquels peut se trouver le système physique, la loi de probabilité conditionnelle de l'observation de chaque état caché étant modélisée par une somme de lois normales.

De façon optionnelle également, le calibrage comporte :

- le calcul de la valeur V de vraisemblance de la séquence d'observation au vu dudit modèle statistique sous la forme :

$$V = \frac{1}{N} \log\left( p\left( O_{1:N} \right) \right),$$

où N est le nombre d'échantillons d'observation dans la séquence d'observation, $O_{1:N}$ désignant les N échantillons successifs,

- une comparaison de cette valeur de vraisemblance à un seuil prédéterminé, ce seuil étant défini à partir du tirage aléatoire d'une pluralité de séquences théoriques d'observation sur la base dudit modèle statistique, et
- le déclenchement de l'estimation d'une nouvelle valeur de référence du vecteur représentatif de l'axe de référence uniquement si la valeur de vraisemblance est inférieure au seuil prédéterminé.

De façon optionnelle également :

- au moins une composante de la séquence d'observation est calculée indépendamment de la valeur de référence du vecteur représentatif de l'axe de référence,

- au moins un état du système physique correspondant à une séquence de mesures est détecté sur la base de cette composante de la séquence d'observation indépendante de la valeur de référence,
- la nouvelle valeur de référence est estimée à partir de la séquence de mesures correspondant à cet état détectable sur la base de la composante de la séquence d'observation indépendante de la valeur de référence.

De façon optionnelle également, l'estimation d'une nouvelle valeur de référence du vecteur représentatif de l'axe de référence comporte les étapes consistant à :

- détecter et récupérer, sur la base de ladite au moins une composante de séquence d'observation indépendante de la valeur de référence, une séquence de mesures correspondant à un état prédéterminé dans lequel le vecteur représentatif de l'axe de référence doit avoir en moyenne la valeur de référence compte tenu dudit modèle statistique,
- calculer les valeurs moyennes des composantes de ce vecteur représentatif dans cette séquence de mesures correspondant à l'état prédéterminé, par exemple par décimation éventuelle de la séquence de mesures correspondant à l'état prédéterminé, filtrage passe bas avec une fréquence de coupure prédéterminée, par exemple 0,125 Hz, de chaque composante de cette séquence de mesures éventuellement décimée, puis calcul des moyennes des composantes filtrées, et
- affecter ces valeurs moyennes à la nouvelle valeur de référence.

L'invention a également pour objet l'application d'un procédé de détection tel que défini précédemment à l'observation, à l'aide d'un accéléromètre tridimensionnel, d'une personne pouvant se trouver à chaque instant dans l'un des états prédéterminés suivants : un état de « position assise », un état de « transition assis/debout », un état de « position debout statique », un état de « marche » et un état de « transition debout/assis » ; la valeur de référence étant la valeur que prennent, dans le repère des axes de mesure de l'accéléromètre tridimensionnel, les composantes d'un vecteur unitaire, vertical et orienté vers le bas lorsque la personne observée porte l'accéléromètre tridimensionnel et est dans l'état de « position debout statique » ou de « marche ».

L'invention a également pour objet un dispositif de détection de l'activité d'un système physique comportant :

- un capteur de mouvements destiné à être porté par le système physique pour la fourniture d'une séquence de mesures,
- des moyens de stockage d'un modèle statistique associant des valeurs possibles de composantes d'une séquence d'observation à une pluralité d'états prédéterminés possibles dans lesquels peut se trouver le système physique,
- un calculateur, relié au capteur de mouvements et aux moyens de stockage, programmé pour calculer une séquence d'observation à partir de la séquence de mesures et pour déterminer, à l'aide du modèle statistique, l'activité du système physique sous la forme d'une séquence d'états correspondant à la séquence d'observation,

dans lequel le calculateur est programmé pour calculer au moins une composante de la séquence d'observation à partir d'une valeur de référence d'un vecteur représentatif d'un axe de référence, le dispositif comportant des moyens de calibrage conçus pour :

- calculer une valeur de vraisemblance de la séquence d'observation au vu dudit modèle statistique,
- comparer cette valeur de vraisemblance à un seuil prédéterminé,
- déclencher une estimation d'une nouvelle valeur de référence du vecteur représentatif de l'axe de référence en fonction de cette comparaison.

De façon optionnelle, le capteur de mouvements est un accéléromètre à trois axes de mesure, l'axe de référence estimable à l'aide des mesures fournies par le capteur de mouvements étant l'axe du champ de gravitation terrestre, la valeur de référence d'un vecteur représentatif de cet axe étant calculée à partir d'une estimation des coordonnées d'une accélération due au champ de gravitation terrestre dans le référentiel des axes de mesure de l'accéléromètre lorsque le système physique est dans un état prédéterminé.

L'invention a également pour objet un système de détection de l'activité d'un système physique, comportant :

- une ceinture destinée à être portée au torse ou à la taille d'une personne, et
- un boîtier fixé à la ceinture et comportant un dispositif de détection tel que défini précédemment.

Enfin, l'invention a également pour objet un programme d'ordinateur téléchargeable depuis un réseau de communication et/ou enregistré sur un support lisible par ordinateur et/ou exécutable par un processeur, comprenant des instructions pour l'exécution des étapes d'un procédé de détection tel que défini précédemment, lorsque ledit programme est exécuté sur un ordinateur.

L'invention sera mieux comprise à l'aide de la description qui va suivre, donnée uniquement à titre d'exemple et faite en se référant aux dessins annexés dans lesquels :

- la figure 1 représente schématiquement la structure générale d'un dispositif de détection selon un mode de réalisation de l'invention,
- la figure 2 illustre une utilisation particulière du dispositif de détection de la figure 1,
- la figure 3 illustre un exemple de modèle statistique de Markov à états cachés présentant des contraintes de transitions entre états cachés et pouvant être pris en compte par le dispositif de détection de la figure 1,
- la figure 4 illustre les étapes successives d'un procédé de détection selon un mode de réalisation de l'invention,
- la figure 5 illustre le détail d'une étape de calibrage du procédé de la figure 4,
- la figure 6 illustre les étapes successives d'un procédé de calcul d'un paramètre de seuil pris en compte dans le procédé de la figure 4, et
- la figure 7 illustre un ensemble de trois diagrammes temporels de données intermédiaires ou de sortie du dispositif de la figure 1 mettant en oeuvre le procédé de la figure 4.

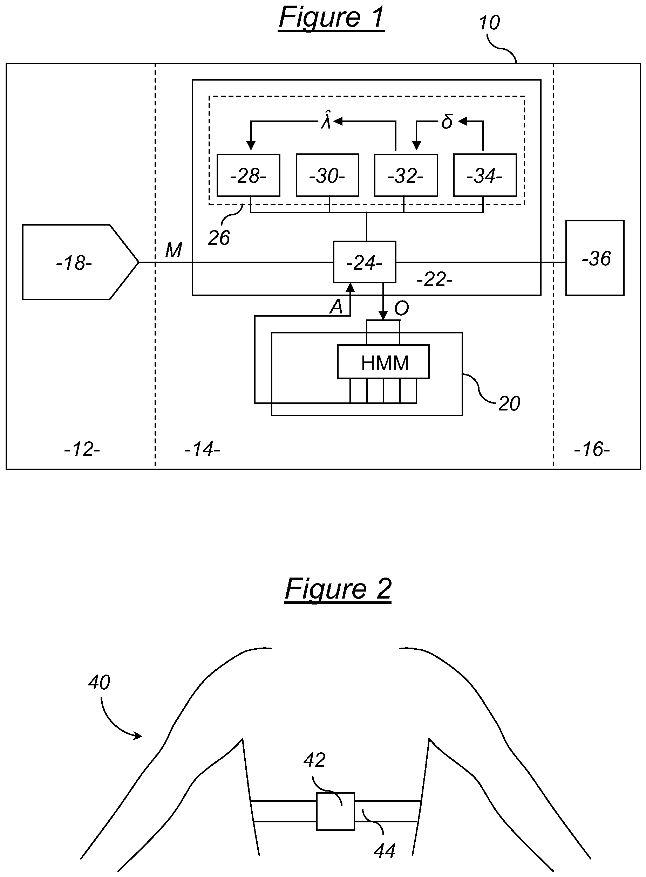

Le dispositif 10 représenté sur la figure 1 est un dispositif de détection de l'activité d'un système physique observé par au moins un capteur. Il comporte à cet effet un module de mesure 12, un module de traitement 14 et un module d'interface 16.

Le module de mesure 12 comporte un ou plusieurs capteurs, dont au moins un capteur de mouvements, représentés par l'unique référence 18 pour l'observation du système physique.

De façon non limitative, le capteur 18 peut par exemple comporter un capteur de mouvements à un, deux ou trois axes de mesure, notamment un accéléromètre tridimensionnel porté par une personne, pour la détermination de l'activité de cette personne sous la forme d'une séquence d'attitudes parmi plusieurs attitudes possibles susceptibles de se succéder (par exemple assis, debout, transition assis/debout ou debout/assis, marche).

Plus généralement, il peut comporter un capteur de mouvements pour la détermination de l'activité d'un système physique mobile sous la forme d'une séquence d'états parmi plusieurs états prédéterminés susceptibles de se succéder.

Le capteur de mouvements 18 peut aussi comporter plusieurs capteurs fournissant chacun des mesures qui, combinées, permettent d'envisager de détecter des situations plus complexes.

Il effectue des mesures sur le système physique pour fournir un signal de mesure, transmis sous la forme d'une séquence M de données de mesure au module de traitement 14. Les données de mesure sont directement issues d'un échantillonnage du signal de mesure, par exemple à 100 ou 200 Hz. Dans le cas d'un accéléromètre tridimensionnel, chaque échantillon de données de mesure est un vecteur à trois composantes, chaque composante représentant la projection de l'accélération totale mesurée par le capteur sur l'un de ses trois axes de mesure. Cette accélération totale comporte l'accélération de gravitation terrestre et l'accélération propre du capteur de mouvements 18.

Le capteur de mouvements 18 présente également la propriété de permettre, à l'aide des mesures qu'il fournit, d'estimer un axe de référence représenté par un vecteur de référence $\lambda$, par exemple de norme 1, indiquant sa direction et son sens. Comme cela sera détaillé par la suite, les coordonnées de ce vecteur de référence peuvent être exprimées dans le référentiel du capteur de mouvements 18 et prendre une valeur particulière de référence $\hat{\lambda} = (\hat{\lambda}_x, \hat{\lambda}_y, \hat{\lambda}_z)$ lorsque le capteur de mouvements 18 est dans une orientation prédéterminée et privilégiée correspondant par exemple à au moins l'un des états dans lequel le système physique peut se trouver. Dans le cas d'un capteur accélérométrique porté par une personne, l'axe de référence est avantageusement l'axe du champ de gravitation terrestre.

Le module de traitement 14 est un circuit électronique, notamment celui d'un ordinateur. Il comporte des moyens de stockage 20, par exemple une mémoire de type RAM, ROM ou autre, dans lesquels sont stockés les paramètres d'au moins un modèle statistique, par exemple un modèle HMM de Markov à état cachés.

D'une façon générale, ce modèle statistique stocké en mémoire 20, ou l'ensemble structuré de ces modèles statistiques s'il y en a plusieurs combinés, associe une succession de valeurs possibles de données d'observation à une succession d'états correspondant à au moins une partie des états cachés du modèle statistique HMM et identifiant les états dans lesquels peut se trouver le système physique portant le capteur de mouvements 18.

Le module de traitement 14 comporte en outre un calculateur 22, par exemple une unité centrale d'ordinateur munie d'un microprocesseur 24 et d'un espace de stockage d'au moins un programme d'ordinateur 26. Ce calculateur 22, et plus particulièrement le microprocesseur 24, est relié au capteur de mouvements 18 et à la mémoire 20.

Le programme d'ordinateur 26 remplit quatre fonctions principales illustrées par des modules 28, 30, 32 et 34 sur la figure 1.

La première fonction principale, remplie par un module de filtrage 28, est une fonction de transformation de la séquence de mesures M fournie par le capteur de mouvements 18 en une séquence d'observation O pouvant être confrontée au modèle statistique HMM stocké en mémoire 20. Comme la séquence de mesures M, la séquence d'observation O est formée d'une succession d'échantillons, chacun de ces échantillons pouvant être un vecteur dont chaque composante est issue d'un traitement particulier de la séquence de mesures M. D'une façon générale, conformément à l'invention, au moins une composante de chaque échantillon de la séquence d'observation O est calculée à partir de la valeur de référence $\hat{\lambda}$.

A titre d'exemple non limitatif, lorsque le capteur de mouvements 18 est un accéléromètre tridimensionnel porté par une personne dont on souhaite déterminer l'activité sous la forme d'une séquence d'attitudes prédéterminées possibles dans lesquelles elle peut se trouver (parmi : position assise, transition assis/debout, position debout statique, marche, transition debout/assis), la séquence M est une séquence de vecteurs d'accélération $\gamma_n = \gamma_n^g + \gamma_n^p$, désignant l'indice temporel des échantillons, $\gamma_n^g$ la partie basse fréquence de ces vecteurs d'accélération correspondant à l'accélération due au champ de gravitation terrestre pouvant être obtenue par un simple filtrage passe bas et $\gamma_n^p$ la partie haute fréquence de ces vecteurs d'accélération correspondant à l'accélération propre du capteur pouvant être obtenue par un simple filtrage passe bande. Ces vecteurs sont connus par leurs composantes projetées sur les trois axes de mesure du capteur de mouvements 18.

Le module de filtrage 28 peut ainsi séparer ces deux parties $\gamma_n^g$ et $\gamma_n^p$ et fournir une séquence d'observation O à deux composantes :

- une première séquence de composantes $O_n(1)$, obtenue par décimation à 5 Hz de la séquence de mesure M, filtrage passe bas des échantillons résultants avec une fréquence de coupure de 0,125 Hz, puis projection normalisée du vecteur $\gamma_n^g$ ainsi obtenu sur la valeur de référence $\hat{\lambda}$,
- une seconde séquence de composantes $O_n(2)$, obtenue par décimation à 5 Hz de la séquence de mesure M, filtrage passe bande des échantillons résultants avec une fréquence de coupure basse de 0,125 Hz et une fréquence de coupure haute de 0,75 Hz, puis calcul de la norme du vecteur $\gamma_n^g$ ainsi obtenu.

$$O_n(1) = 100 \cdot \frac{\left\langle \gamma_n^g, \hat{\lambda} \right\rangle}{\left\| \gamma_n^g \right\|},$$

Plus précisément, et à titre d'exemple : où $\langle,\rangle$ désigne le produit scalaire et $\|\|$ la norme euclidienne, $O_n(2) = 100 \cdot \left\| \gamma_n^p \right\|$.

Ainsi, on remarque que la première composante $O_n(1)$ de chaque échantillon de la séquence d'observation O est calculée à partir de la valeur de référence $\hat{\lambda}$ alors que la seconde composante $O_n(2)$ est indépendante de cette valeur de référence.

La deuxième fonction principale, remplie par un module de détection d'activité 30, par exemple sous la forme d'une boucle d'instructions, est une fonction de détection d'une séquence d'états prédéterminés dans lesquels peut se trouver le système physique, à partir d'une séquence O de données d'observation fournie par le module de filtrage 28. Plus précisément, le module de détection d'activité 30 est programmé pour permettre au microprocesseur 24 de fournir une activité A sous la forme d'une séquence d'états par traitement de la séquence d'observation O à l'aide du modèle statistique HMM stocké en mémoire 20.

La troisième fonction principale, remplie par un module de test 32, est une fonction de calcul d'une valeur de vraisemblance de la séquence d'observation O au vu du modèle HMM stocké en mémoire 20 et de comparaison de cette valeur de vraisemblance à un seuil δ pour une éventuelle mise à jour de la valeur de référence $\hat{\lambda}$.

Enfin, la quatrième fonction principale, remplie par un module de calcul de seuil 32, est une fonction de calcul de la valeur seuil δ pour le modèle HMM considéré.

Le module d'interface 16 comporte une interface 36 permettant d'afficher le résultat d'une détection réalisée par le microprocesseur 24, par exemple sous la forme d'un diagramme temporel des états successifs correspondant à l'observation O. Un des intérêts possibles du dispositif de détection 10 étant éventuellement en outre de détecter une situation critique prédéterminée, cette interface 36 peut notamment comporter un déclencheur d'alerte. L'interface 36 ou son déclencheur d'alerte peut par exemple comporter un écran, pour l'affichage de résultats ou d'un message d'avertissement, un haut-parleur, pour l'émission d'un signal sonore, ou un émetteur, pour la transmission d'un signal vers une alarme distante. Le déclencheur d'alerte peut être activé sur détection d'une ou plusieurs activité(s) particulière(s). Un exemple d'événement/activité détectable susceptible de déclencher une alerte est par exemple une chute.

On notera que les modules d'observation 12, de traitement 14 et d'interface 16 sont structurellement séparables. Ainsi le dispositif de détection 10 peut être conçu d'un seul tenant ou en plusieurs éléments matériels distincts reliés entre eux par des moyens de transmission de données avec ou sans fil. En particulier, les modules de traitement 14 et éventuellement d'interface 16 peuvent être mis en oeuvre par ordinateur. Seul le module d'observation 12 est nécessairement au voisinage voire au contact du système physique observé puisqu'il comporte le ou les capteurs 18.

Sur la figure 2, un mode de réalisation particulièrement compact est illustré, pour une application de surveillance de l'activité d'une personne 40. Selon ce mode de réalisation, le dispositif de détection 10 de la figure 1 est tout entier intégré dans un boîtier 42 porté par la personne 40. Le capteur est un accéléromètre tridimensionnel et les attitudes successives caractéristiques de l'activité de la personne 40 sont par exemple au nombre de cinq : une attitude de « position assise », une attitude de « transition assis/debout », une attitude de « position debout statique », une attitude de « marche » et une attitude de « transition debout/assis », chacune de ces attitudes étant représentée par un état caché correspondant d'un unique modèle de Markov HMM. Pour cette application, le boîtier 42 est par exemple fermement maintenu au torse ou à la taille de la personne 40 au moyen d'une ceinture 44.

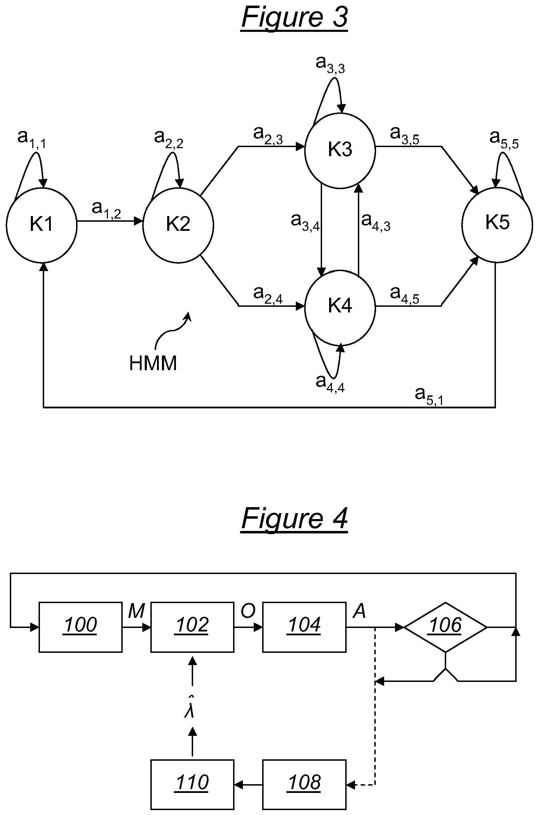

Un exemple de modèle statistique HMM pouvant être stocké en mémoire va maintenant être détaillé en référence à la figure 3, pour l'application mentionnée ci-dessus. Ce modèle HMM est un modèle de Markov à états cachés dont chaque état caché correspond à l'une des cinq attitudes mentionnées ci-dessus. Il est défini par les paramètres suivants :

- le nombre d'états cachés de ce modèle HMM, égal à 5 dans l'exemple de la figure 3,
- $\pi_1$, ..., $\pi_5$, les cinq probabilités initiales, indépendantes de toute observation, de chaque état caché de ce modèle HMM, par exemple toutes égales à 0,2,
- $(a_{i,j})_{1 \leq i,j \leq 5}$, la matrice des probabilités de transition de chaque état caché i vers chaque autre état caché j de ce modèle HMM, et
- pour chaque état caché, les paramètres d'une loi de probabilité de l'observation fournie à chaque instant en entrée de ce modèle.

Les états cachés du modèle HMM de la figure 3 sont ainsi les suivants : K1 pour l'état de « position assise », K2 pour l'état de « transition assis/debout », K3 pour l'état de « position debout statique », K4 pour l'état de « marche » et K5 pour l'état de « transition debout/assis ».

On note par ailleurs que ce modèle impose certaines contraintes de transitions impossibles, telles que par exemple la transition de l'état de « position debout statique » à l'état de « position assise » qui ne peut se faire directement sans passer par l'état de « transition debout/assis ». Il en résulte par exemple la matrice des probabilités de transition

$$\left(a_{i,j}\right)_{5x5} = \begin{pmatrix} 1-\varepsilon & \varepsilon & 0 & 0 & 0 \\ 0 & 1-\varepsilon & \varepsilon/2 & \varepsilon/2 & 0 \\ 0 & 0 & 1-\varepsilon & \varepsilon/2 & \varepsilon/2 \\ 0 & 0 & \varepsilon/2 & 1-\varepsilon & \varepsilon/2 \\ \varepsilon & 0 & 0 & 0 & 1-\varepsilon \end{pmatrix},$$

suivante : où la variable $\varepsilon$ est par exemple comprise entre $10^{-2}$ et $10^{-4}$.

Sur la figure 3, seules les probabilités de transition non nulles sont représentées.

Les lois de probabilité de l'observation à chaque instant et pour chaque état caché sont définies par des paramètres qui sont bien sûr dépendants de la valeur de référence $\hat{\lambda}$ arbitrairement choisie. Par exemple, dans l'application détaillée ci-dessus, le vecteur de référence $\lambda$ est un vecteur unitaire vertical et orienté vers le bas dans le référentiel terrestre, la valeur de référence $\hat{\lambda}$ étant la valeur que prennent les composantes de ce vecteur de référence $\lambda$ dans le repère des axes de mesure de l'accéléromètre tridimensionnel lorsqu'il est porté par la personne observée et lorsque celle-ci est en état K3 de « position debout statique ». En choisissant une telle valeur de référence $\hat{\lambda}$, il est possible de l'initialiser à une première valeur obtenue par étalonnage, en prenant une série de mesures lorsque la personne observée porte le capteur de mouvements 18 et est en position debout statique. $\hat{\lambda}$ correspond en effet alors au vecteur d'accélération mesuré qu'il suffit de moyenner sur la série mesurée. De façon plus générale, il est avantageux de choisir une valeur de référence qui puisse être déduite directement des mesures associé à l'un des états prédéterminés. On notera également qu'il est possible d'initialiser $\hat{\lambda}$ à une valeur arbitraire sans étalonnage.

L'une quelconque des lois de probabilité conditionnelle de l'observation connaissant l'état à déterminer peut être choisie dans la famille des lois normales. Une loi normale est définie par son espérance $\mu$ et sa variance $\Sigma$. Lorsque les données fournies en entrée du modèle HMM sont multivaluées, $\mu$ est un vecteur comportant autant de composantes et $\Sigma$ une matrice comportant autant de lignes et de colonnes que de valeurs fournies à chaque instant en entrée du modèle HMM.

Dans l'exemple précité, la séquence d'observation étant à deux composantes, $\mu$ est un vecteur à deux composantes et $\Sigma$ une matrice 2x2. Dans l'exemple précité également, chaque loi de probabilité conditionnelle de l'observation connaissant l'état à déterminer est en pratique avantageusement une somme de deux lois normales pouvant s'écrire

$$p\left(O_n \middle| A_n = i\right) = \sum_{j=1}^{2} c_{i,j} \frac{1}{\sqrt{2\pi}^{3/2} \left|\Sigma_{i,j}\right|^{1/2}} \exp\left(-\frac{1}{2}\left(O_n - \mu_{i,j}\right)^{T} \Sigma_{i,j}^{-1}\left(O_n - \mu_{i,j}\right)\right),$$

sous la forme :                                                                                                                   où les

paramètres $c_{i,j}$, $\mu_{i,j}$ et $\Sigma_{i,j}$ sont par exemple définis comme indiqué par le tableau suivant :

| Etat Ki | $c_{i,1}$ | $\mu_{i,1}$ | $\Sigma_{i,1}$ | $c_{i,2}$ | $\mu_{i,2}$ | $\Sigma_{i,2}$ |
|---|---|---|---|---|---|---|
| i = 1 | 0,424 | $\begin{bmatrix} 78,28 \\ 0,0059 \end{bmatrix}$ | $\begin{bmatrix} 17,72 & 0,024 \\ 0,024 & 0,0177 \end{bmatrix}$ | 0,576 | $\begin{bmatrix} 95,072 \\ 0,0085 \end{bmatrix}$ | $\begin{bmatrix} 16,40 & -0,016 \\ -0,016 & 0,0164 \end{bmatrix}$ |
| i = 2 | 0,308 | $\begin{bmatrix} 85,26 \\ 1,5033 \end{bmatrix}$ | $\begin{bmatrix} 71,25 & 4,99 \\ 4,99 & 4,36 \end{bmatrix}$ | 0,692 | $\begin{bmatrix} 99,37 \\ 0,461 \end{bmatrix}$ | $\begin{bmatrix} 0,496 & -0,155 \\ -0,155 & 0,51 \end{bmatrix}$ |
| i = 3 | 0,950 | $\begin{bmatrix} 99,73 \\ 0,0044 \end{bmatrix}$ | $\begin{bmatrix} 0,078 & 0,000067 \\ 0,000067 & 0,0001 \end{bmatrix}$ | 0,050 | $\begin{bmatrix} 99,82 \\ 0,137 \end{bmatrix}$ | $\begin{bmatrix} 0,016 & -0,00045 \\ -0,00045 & 0,0135 \end{bmatrix}$ |
| i = 4 | 0,083 | $\begin{bmatrix} 99,33 \\ 1,61 \end{bmatrix}$ | $\begin{bmatrix} 0,133 & -0,09 \\ -0,09 & 2,69 \end{bmatrix}$ | 0,916 | $\begin{bmatrix} 99,9 \\ 1,567 \end{bmatrix}$ | $\begin{bmatrix} 0,0098 & 0,021 \\ 0,021 & 2,36 \end{bmatrix}$ |
| i = 5 | 0,190 | $\begin{bmatrix} 93,62 \\ 3,04 \end{bmatrix}$ | $\begin{bmatrix} 57,98 & -2,44 \\ -2,44 & 4,16 \end{bmatrix}$ | 0,810 | $\begin{bmatrix} 93,31 \\ 0,22 \end{bmatrix}$ | $\begin{bmatrix} 48,61 & -0,02 \\ -0,02 & 0,08 \end{bmatrix}$ |

Le fonctionnement du dispositif de détection 10 va maintenant être détaillé en référence à la figure 4. Plus précisément, l'exécution du module de filtrage 28, du module de détection d'activité 30 et du module de test 32 par le microprocesseur 24 produit la séquence d'étapes illustrées sur cette figure.

Au cours d'une première étape 100, le capteur de mouvements 18, en l'occurrence l'accéléromètre tridimensionnel, transmet une séquence M de données de mesures au calculateur 22. Ces données sont vectorielles et à trois composantes indicatrices de la projection de l'accélération mesurée sur chaque axe de mesure du capteur de mouvements 18.

Au cours d'une étape 102, pendant la réception 100 des données successives transmises par le capteur de mouvements 18, le microprocesseur 24 du calculateur 22 lance l'exécution du module de filtrage 28 comme décrit précédemment, pour la production d'une séquence de N données d'observation notée $O_{1:N} = \{O_1,...,O_N\}$, dans laquelle chaque échantillon $O_n$ est un vecteur à deux composantes $O_n(1)$ et $O_n(2)$ telles que définies précédemment.

Au cours de l'étape 104 suivante, le microprocesseur 24 du calculateur 22 lance l'exécution du module logiciel de détection d'activité 30. A l'aide du modèle statistique HMM précité, l'activité de la personne portant le capteur de mouvements 18 est déterminée sous la forme d'une séquence d'états (ou séquence d'attitudes) correspondant à la séquence d'observation $O_{1:N}$. Cette séquence d'états est notée $\hat{A}_{1:N} = \{\hat{A}_1,..,\hat{A}_N\}$, dans laquelle chaque échantillon $\hat{A}_n$ est un entier naturel compris entre 1 et 5 identifiant l'un des cinq états du modèle (« position assise », « transition assis/debout », « position debout statique », « marche », « transition debout/assis »). Compte tenu du modèle HMM de Markov à états cachés choisi :

$$\begin{aligned} \hat{A}_{1:N} &= \arg\max_{A_{1:N}}\left[p\left(A_{1:N}\middle|O_{1:N}\right)\right] \\ &= \arg\max_{A_{1:N}}\left[p\left(A_{1:N},O_{1:N}\right)/p\left(O_{1:N}\right)\right], \\ &= \arg\max_{A_{1:N}}\left[p\left(A_{1:N},O_{1:N}\right)\right] \end{aligned}$$

avec :

$$p(A_{1:N}, O_{1:N}) = p(A_1)p(O_1|A_1)\prod_{n=2}^{N} p(A_n|A_{n-1})p(O_n|A_n).$$

De façon classique, la séquence d'états $\hat{A}_{1:N}$ est estimée à l'aide d'un algorithme de Viterbi.

Ensuite, au cours d'une étape 106, le microprocesseur 24 du calculateur 22 lance l'exécution du module de test 32 pour le calcul d'une valeur V de vraisemblance de la séquence d'observation $O_{1:N}$ au vu du modèle statistique HMM. La valeur de vraisemblance V est par exemple donnée par la formule suivante : $V = \dfrac{1}{N}\log\big(p(O_{1:N})\big),$ dans laquelle l'expression $p(O_{1:N})$ se développe sous la forme :

$$p(O_{1:N}) = \sum_{A_1=1}^{M}\ldots\sum_{A_N=1}^{M} p(O_{1:N}, A_{1:N})$$
$$= \sum_{A_1=1}^{M}\ldots\sum_{A_N=1}^{M} p(A_1)p(O_1|A_1)\prod_{n=2}^{N} p(A_n|A_{n-1})p(O_n|A_n)$$

où M est le nombre d'états, soit 5 dans l'exemple considéré. N est par exemple égal à 10 ou 20.

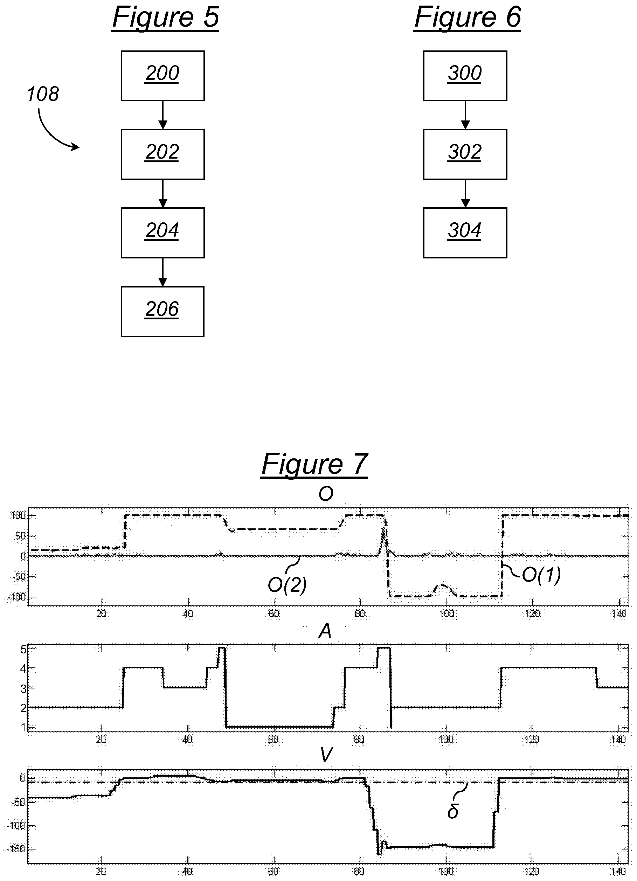

Au vu des lois de probabilités précités, cette valeur de vraisemblance V est calculable directement sans difficulté particulière. Elle est d'autant plus élevée que la valeur de référence $\hat{\lambda}$ reste adaptée à l'observation en cours. En effet, la situation peut avoir évolué depuis l'initialisation de la valeur de référence ou depuis sa dernière mise à jour, du fait d'un changement de position du capteur accélérométrique sur le corps de la personne observée, du fait d'un changement de personne observée ou du fait d'un autre évènement perturbateur. Dans ce cas, la valeur de vraisemblance V peut se dégrader. Il est donc pertinent de vérifier régulièrement que cette valeur reste supérieure à un seuil prédéterminé noté $\delta$. Ce seuil $\delta$ est dépendant uniquement du modèle HMM stocké en mémoire 20 et un procédé pour le calcul de sa valeur sera détaillé en référence à la figure 6. Compte tenu des paramètres numériques du modèle HMM détaillé précédemment, le procédé illustré sur la figure 6 fournit la valeur $\delta$ = - 8.

Au cours de l'étape 106, la valeur de vraisemblance V est comparée à la valeur seuil $\delta$. Si V>$\delta$, le procédé retourne directement à l'étape 100 pour le traitement d'une nouvelle séquence de mesures. Sinon, le procédé déclenche une étape 108 de mise à jour de la valeur de référence $\hat{\lambda}$ avant de retourner à l'étape 100.

L'étape de mise à jour 108 s'exécute parallèlement à la boucle d'étapes 100 à 106 et se base sur les séquences d'états successives fournies en sortie de l'étape 104. Dans l'exemple considéré, elle se base sur la détection d'un état dans lequel une accélération propre suffisante est mesurée pendant une durée minimale. En effet, cette accélération propre est détectée par la seconde composante des échantillons de la séquence d'observation qui est indépendante de la valeur de référence $\hat{\lambda}$ et donc insensible à ses dérives. En outre, en imposant une durée minimale, par exemple de 12 secondes, on distingue l'état de marche des états de transition assis/debout ou transition debout/assis qui ne durent pas aussi longtemps et qui sont les seuls, avec l'état de marche, à présenter une accélération propre suffisante. Ainsi, indépendamment de la valeur de référence $\hat{\lambda}$, il est possible de détecter précisément lorsque la personne observée est dans l'état de marche. Or cet état de marche est également un état dans lequel, comme dans l'état de position debout statique, la partie basse fréquence du vecteur d'accélération mesuré doit être en moyenne colinéaire au vecteur de référence $\lambda$ pour que le modèle statistique s'applique de façon optimale, comme cela a été défini par construction du modèle.

Ainsi, l'étape 108 consiste, comme illustré sur la figure 5, à :

- détecter (200) une séquence de données d'observation successives, pendant laquelle une accélération propre suffisante est mesurée sur une durée supérieure à une durée minimale prédéfinie (par exemple 12 secondes),
- récupérer (202) la séquence de mesures correspondante, tronquer cette séquence de quelques secondes au début et à la fin pour être sûr de ne conserver qu'une séquence de marche,
- sur cette séquence de mesures tronquée, réaliser (204) une décimation à 5 Hz, puis un filtrage passe bas des échantillons résultants avec une fréquence de coupure de 0,125 Hz,
- calculer (206) la moyenne sur la séquence tronquée de chaque composante vectorielle mesurée et filtrée et affecter cette moyenne à la composante correspondante de la valeur de référence $\hat{\lambda}$ .

Pour détecter une accélération propre suffisante (évènement noté par exemple ACC, l'évènement contraire étant alors noté $\overline{\text{ACC}}$), on peut astucieusement définir deux nouvelles lois de probabilité a posteriori pour la seconde composante d'observation $O_n(2)$ :

$$p\big(O_n(2)\big|ACC\big) = \frac{1}{\sqrt{2\pi}^{\,1/2}\,\sigma_{ACC}^{\,1/2}}\exp\left(-\frac{\big(O_n(2)-\mu_{ACC}\big)^2}{2\,\sigma_{ACC}^{\,2}}\right),$$

avec par exemple

$$\mu_{ACC} = 1{,}282134 \text{ et } \sigma_{ACC} = 1{,}09\,,$$

et

$$p\big(O_n(2)\big|\overline{ACC}\big) = \frac{1}{\sqrt{2\pi}^{\,1/2}\,\sigma_{\overline{ACC}}^{\,1/2}}\exp\left(-\frac{\big(O_n(2)-\mu_{\overline{ACC}}\big)^2}{2\,\sigma_{\overline{ACC}}^{\,2}}\right),$$

avec par exemple

$$\mu_{\overline{ACC}} = 0{,}008 \text{ et } \sigma_{\overline{ACC}} = 0{,}045\,.$$

On décide alors à chaque instant (c'est-à-dire pour chaque échantillon) que l'accélération propre est suffisante si :

$$\frac{p\big(O_n(2)\big|ACC\big)}{p\big(O_n(2)\big|ACC\big) + p\big(O_n(2)\big|\overline{ACC}\big)} > 0{,}8\,.$$

Il faut donc que cette condition soit vérifiée pendant au moins 12 secondes pour que la mise à jour de la valeur de référence $\hat{\lambda}$ puisse être réalisée.

L'étape 108 est optionnellement mais avantageusement suivie d'une étape 110 d'invalidation de l'activité déterminée, au moins partiellement par invalidation d'au moins une partie de la séquence d'états déterminée à partir de la séquence d'observation à l'étape 104. Plus précisément, les états déterminés pour lesquels la valeur de vraisemblance est inférieure à la valeur du seuil δ peuvent être invalidés. De façon optionnelle également, il peut être généré une information permettant de prévenir l'utilisateur qu'une activité a été déterminée alors que la valeur de vraisemblance était faible. Cette information peut être affichée par l'interface 36.

Le procédé de calcul de la valeur du seuil δ, illustré sur la figure 6, comporte une première étape 300 lors de laquelle on procède au tirage aléatoire d'un grand nombre de séquences théoriques d'observation sur la base du modèle d'observation défini précédemment, par exemple au moins 1000. Concrètement, pour réaliser ce tirage aléatoire, pour chaque séquence théorique d'observation on tire aléatoirement un premier état initial $A_1$, puis on tire aléatoirement une valeur d'observation selon la loi $p(O|A_1)$. Ensuite on procède de manière itérative jusqu'à la N-ième valeur d'observation en tirant aléatoirement l'état suivant selon les lois de transition définies pour le modèle HMM, puis la valeur d'observation suivante selon la loi qui correspond à la réalisation de cet état suivant.

Ensuite, au cours d'une étape 302, on calcule la valeur de vraisemblance V pour chacune des séquences théoriques d'observation obtenues à l'étape 300.

Enfin, au cours d'une dernière étape 304, on détermine la valeur de δ de telle sorte que, par exemple, 95% des valeurs de vraisemblance V calculées à l'étape 302 soient supérieures à cette valeur δ. En particulier, comme indiqué précédemment, cela donne δ = - 8 pour le modèle détaillé précédemment et illustré sur la figure 3.

Trois exemples de diagrammes temporels de données intermédiaires ou de sortie du dispositif de la figure 1 mettant en oeuvre le procédé de détection de la figure 4 sont illustrés sur la figure 7.

Le premier diagramme fournit un exemple O de séquence de données d'observation à deux composantes telles que définies précédemment. La séquence des premières composantes $O_n(1)$ est notée O(1) et la séquence des secondes composantes $O_n(2)$ est notée O(2).

Le deuxième diagramme fournit l'activité A résultante estimée par le module de détection d'activité 30 sous forme d'une séquence d'états : en ordonnée de ce diagramme, 1 identifie l'état de position assise (état caché K1), 2 l'état de transition assis/debout (état caché K2), 3 l'état de position debout statique (état caché K3), 4 l'état de marche (état caché K4) et 5 l'état de transition debout/assis (état caché K5).

Le troisième diagramme fournit une séquence résultante de valeurs de vraisemblance V, estimée par le module de test 32 sur des fenêtres glissantes. Les valeurs de vraisemblance V sont comparées à chaque instant au seuil δ.

On remarque, sur les premiers et troisièmes diagrammes, que jusqu'au 25e échantillon environ, la valeur de référence $\hat{\lambda}$ semble être mal adaptée au modèle puisqu'un état de transition assis/debout est détecté pendant une durée trop longue, ce que confirme la valeur de vraisemblance V < δ.

Suite à la détection d'une période suffisamment longue d'accélération propre mesurée entre les 15e et 25e échantillons (cf. courbe O(2)), la valeur de référence $\hat{\lambda}$ est mise à jour vers le 25e échantillon, ce que confirme la valeur de vraisemblance V > δ à partir de cet instant.

On remarque alors que suite à une autre période d'accélération propre entre les 75e et 80e échantillons, au cours de laquelle le capteur de mouvements 18 a dû être malencontreusement déplacé, la valeur de vraisemblance passe de nouveau sous le seuil δ.

La situation est rétablie vers le 112e échantillon, suite à la détection d'une nouvelle période suffisamment longue d'accélération propre (cf. courbe O(2)), lorsque la valeur de référence $\hat{\lambda}$ est de nouveau mise à jour, ce que confirme la valeur de vraisemblance V > δ à partir de cet instant.

On note que les décisions prises alors que V < δ pourraient en variante ne pas être indiquées dans la séquence d'activité A. On note également que cette séquence d'activité A pourrait être directement affichée, ou représentée d'une façon ou d'une autre, sur l'interface 36.

Il apparaît clairement qu'un dispositif de détection tel que celui décrit précédemment est capable de détecter des dérives dans l'estimation des états observés et de s'autocalibrer en conséquence. Il est donc plus robuste que les dispositifs similaires de l'état de l'art.

On notera par ailleurs que l'invention n'est pas limitée au mode de réalisation décrit précédemment.

En particulier, la valeur de référence $\hat{\lambda}$ n'est pas nécessairement celle qui a été définie précédemment, à savoir la valeur que prennent, dans le repère des axes de mesure de l'accéléromètre tridimensionnel 18, les composantes d'un vecteur unitaire, vertical et orienté vers le bas lorsque la personne observée porte l'accéléromètre tridimensionnel et est dans l'état de « position debout statique ». Cette valeur de référence est bien entendu très liée à l'application choisie comme exemple non limitatif.

D'une façon plus générale, la valeur de référence est la valeur que prennent, dans un repère lié au capteur de mouvements 18, les composantes d'un vecteur de référence estimable à l'aide des mesures fournies par le capteur lorsque le système physique observé est dans un état prédéterminé dans lequel ce vecteur de référence est supposé stable, cet état étant de préférence détectable à partir de composantes d'observation indépendantes de la valeur de référence.

Il apparaîtra également plus généralement à l'homme de l'art que diverses modifications peuvent être apportées aux modes de réalisation décrits ci-dessus, à la lumière de l'enseignement qui vient de lui être divulgué. Dans les revendications qui suivent, les termes utilisés ne doivent pas être interprétés comme limitant les revendications au mode de réalisation exposé dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

**Revendications**

**1.** Procédé de détection de l'activité (A) d'un système physique (40) porteur d'un capteur de mouvements (18), comportant les étapes suivantes exécutées par un calculateur (22):

- extraire (100) une séquence (M) de mesures fournies par le capteur de mouvements (18),
- en déduire (102) une séquence d'observation (O) calculée à partir de la séquence de mesures (M),
- à l'aide d'un modèle statistique (HMM) de composantes de la séquence d'observation (O) au vu d'une pluralité d'états prédéterminés possibles (K1, K2, K3, K4, K5) dans lesquels peut se trouver le système physique (40), détermination (104) de l'activité (A) du système physique sous la forme d'une séquence d'états correspondant à la séquence d'observation (O),

**caractérisé en ce qu'**au moins une composante de la séquence d'observation (O) est calculée à partir d'une valeur de référence ($\hat{\lambda}$) d'un vecteur représentatif d'un axe de référence, et **en ce que** le procédé comporte les étapes suivantes de calibrage exécutées par des moyens de calibrage (26,32) :

- calcul (106) d'une valeur de vraisemblance de la séquence d'observation (O) au vu dudit modèle statistique (HMM),

- comparaison (106) de cette valeur de vraisemblance à un seuil prédéterminé ($\delta$),
- en fonction de cette comparaison (106), déclenchement d'une estimation (108) d'une nouvelle valeur de référence ($\hat{\lambda}$) du vecteur représentatif de l'axe de référence.

2. Procédé de détection selon la revendication 1, dans lequel l'axe de référence est estimé par le calculateur (22) via son vecteur représentatif, à l'aide des mesures fournies par le capteur de mouvements (18).

3. Procédé de détection selon la revendication 1 ou 2, comportant en outre une étape (110) exécutée par le calculateur (22), d'invalidation d'au moins une partie de la séquence d'états correspondant à la séquence d'observation (O), en fonction de la valeur de vraisemblance.

4. Procédé de détection selon l'une quelconque des revendications 1 à 3, dans lequel la séquence d'observation (O) est déduite (102) de la séquence de mesures (M) de la façon suivante :

- calcul d'une première composante (O(1)), à partir d'un filtrage passe bas avec une fréquence de coupure prédéterminée de chaque composante de la séquence de mesures, puis d'une projection des composantes résultantes sur la valeur de référence $\hat{\lambda}$,
- calcul d'au moins une seconde composante (0(2)), à partir d'un filtrage passe bande avec des fréquences de coupure prédéterminées de chaque composante de la séquence de mesures.

5. Procédé de détection selon l'une quelconque des revendications 1 à 4, dans lequel le modèle statistique (HMM) est un unique modèle de Markov à états cachés dont chaque état caché correspond à l'un des états prédéterminés possibles (K1, K2, K3, K4, K5) dans lesquels peut se trouver le système physique (40), la loi de probabilité conditionnelle de l'observation de chaque état caché étant modélisée par une somme de lois normales.

6. Procédé de détection selon l'une quelconque des revendications 1 à 5, dans lequel le calibrage exécuté par les moyens de calibrage (26,32) comporte :

- le calcul (106) de la valeur V de vraisemblance de la séquence d'observation (O) au vu dudit modèle statistique

$$V = \frac{1}{N} \log\big(p(O_{1:N})\big),$$

(HMM) sous la forme :  où N est le nombre d'échantillons d'observation dans la séquence d'observation (O), $O_{1:N}$ désignant les N échantillons successifs et $p(O_{1:N})$ leur probabilité,

- une comparaison (106) de cette valeur de vraisemblance à un seuil prédéterminé ($\delta$), ce seuil étant défini à partir du tirage aléatoire d'une pluralité de séquences théoriques d'observation sur la base dudit modèle statistique (HMM), et
- le déclenchement (106) de l'estimation (108) d'une nouvelle valeur de référence ($\hat{\lambda}$) du vecteur représentatif de l'axe de référence uniquement si la valeur de vraisemblance est inférieure au seuil prédéterminé ($\delta$).

7. Procédé de détection selon l'une quelconque des revendications 1 à 6, dans lequel :

- au moins une composante de la séquence d'observation (O) est calculée par le calculateur (22) indépendamment de la valeur de référence ($\hat{\lambda}$) du vecteur représentatif de l'axe de référence,
- au moins un état du système physique (40) correspondant à une séquence de mesures est détecté par un module de détection d'activité (30), sur la base de cette composante de la séquence d'observation (O) indépendante de la valeur de référence ($\hat{\lambda}$),
- la nouvelle valeur de référence ($\hat{\lambda}$) est estimée par le calculateur (22) à partir de la séquence de mesures correspondant à cet état détectable sur la base de la composante de la séquence d'observation (O) indépendante de la valeur de référence ($\hat{\lambda}$).

8. Procédé de détection selon la revendication 7, dans lequel l'estimation (108) d'une nouvelle valeur de référence ($\hat{\lambda}$) du vecteur représentatif de l'axe de référence comporte les étapes consistant à :

- détecter (200) et récupérer (202), sur la base de ladite au moins une composante de séquence d'observation indépendante de la valeur de référence ($\hat{\lambda}$), une séquence de mesures correspondant à un état prédéterminé dans lequel le vecteur représentatif de l'axe de référence doit avoir en moyenne la valeur de référence compte tenu dudit modèle statistique,

- calculer (204, 206) les valeurs moyennes des composantes de ce vecteur représentatif dans cette séquence de mesures correspondant à l'état prédéterminé, par filtrage passe bas (204) avec une fréquence de coupure prédéterminée de chaque composante de cette séquence de mesures, puis calcul (206) des moyennes des composantes filtrées, et
- affecter ces valeurs moyennes à la nouvelle valeur de référence.

9. Application d'un procédé de détection selon l'une quelconque des revendications 1 à 7, à l'observation, à l'aide d'un accéléromètre tridimensionnel (18), d'une personne (40) pouvant se trouver à chaque instant dans l'un des états prédéterminés suivants: un état (K1) de « position assise », un état (K2) de « transition assis/debout », un état (K3) de « position debout statique », un état (K4) de « marche » et un état (K5) de « transition debout/assis » ; la valeur de référence ( $\hat{\lambda}$ ) étant la valeur que prennent, dans le repère des axes de mesure de l'accéléromètre tridimensionnel (18), les composantes d'un vecteur unitaire, vertical et orienté vers le bas lorsque la personne observée (40) porte l'accéléromètre tridimensionnel et est dans l'état de « position debout statique » ou de « marche ».

10. Dispositif (10) de détection de l'activité (A) d'un système physique (40) comportant :

- un capteur de mouvements (18) destiné à être porté par le système physique (40) pour la fourniture d'une séquence de mesures (M),
- des moyens (20) de stockage d'un modèle statistique (HMM) associant des valeurs possibles de composantes d'une séquence d'observation (O) à une pluralité d'états prédéterminés possibles (K1, K2, K3, K4, K5) dans lesquels peut se trouver le système physique (40),
- un calculateur (22), relié au capteur de mouvements (18) et aux moyens de stockage (20), programmé pour calculer une séquence d'observation (O) à partir de la séquence de mesures (M) et pour déterminer, à l'aide du modèle statistique (HMM), l'activité (A) du système physique (40) sous la forme d'une séquence d'états correspondant à la séquence d'observation,

**caractérisé en ce que** le calculateur (22) est programmé (26, 28) pour calculer au moins une composante (O(1)) de la séquence d'observation (O) à partir d'une valeur de référence ($\hat{\lambda}$) d'un vecteur représentatif d'un axe de référence, et **en ce que** le dispositif (10) comporte des moyens de calibrage (26, 32) conçus pour :

- calculer une valeur de vraisemblance de la séquence d'observation (O) au vu dudit modèle statistique (HMM),
- comparer cette valeur de vraisemblance à un seuil prédéterminé ($\delta$),
- déclencher une estimation d'une nouvelle valeur de référence ($\hat{\lambda}$) du vecteur représentatif de l'axe de référence en fonction de cette comparaison.

11. Dispositif de détection (10) selon la revendication 9, dans lequel le capteur de mouvements (18) est un accéléromètre à trois axes de mesure, le calculateur étant programmé pour estimer l'axe de référence à l'aide des mesures fournies par le capteur de mouvements étant l'axe du champ de gravitation terrestre, la valeur de référence ($\hat{\lambda}$) d'un vecteur représentatif de cet axe étant calculée à partir d'une estimation des coordonnées d'une accélération due au champ de gravitation terrestre dans le référentiel des axes de mesure de l'accéléromètre lorsque le système physique (40) est dans un état prédéterminé.

12. Système de détection de l'activité d'un système physique (40), comportant :

- une ceinture (44) destinée à être portée au torse ou à la taille d'une personne, et
- un boîtier (42) fixé à la ceinture (44) et comportant un dispositif de détection selon la revendication 9 ou 10.

13. Programme d'ordinateur téléchargeable depuis un réseau de communication et/ou enregistré sur un support lisible par ordinateur et/ou exécutable par un processeur, **caractérisé en ce qu'**il comprend des instructions pour l'exécution des étapes d'un procédé de détection selon l'une quelconque des revendications 1 à 8, lorsque ledit programme est exécuté sur un ordinateur.

**Patentansprüche**

1. Verfahren zum Nachweis der Aktivität (A) eines physischen Systems (40) als Träger eines Bewegungssensors (18), aufweisend die folgenden Schritte, die von einem Rechner (22) ausgeführt werden:

- Extrahieren (100) einer Sequenz (M) von Messungen, die von dem Bewegungssensor (18) bereitgestellt werden,
- Ableiten daraus (102) einer Beobachtungssequenz (O), die aus der Sequenz von Messungen (M) berechnet wird,
- mit Hilfe eines statistischen Modells (HMM) von Komponenten der Beobachtungssequenz (O) vor dem Hintergrund einer Vielzahl möglicher vorher festgelegter Zustände (K1, K2, K3, K4, K5), in denen sich das physikalische System (40) befinden kann, Bestimmen (104) der Aktivität (A) des physischen Systems in Form einer Sequenz von Zuständen, die der Beobachtungssequenz (O) entspricht,

**dadurch gekennzeichnet, dass** mindestens eine Komponente der Beobachtungssequenz (O) aus einem Referenzwert $(\hat{\lambda})$ eines für eine Referenzachse repräsentativen Vektors berechnet wird, und dadurch, dass das Verfahren die folgenden Kalibrierungsschritte aufweist, die von Kalibrierungsmitteln (26, 32) ausgeführt werden:

- Berechnen (106) eines Wahrscheinlichkeitswerts der Beobachtungssequenz (O) vor dem Hintergrund des statistischen Modells (HMM),
- Vergleichen (106) dieses Wahrscheinlichkeitswerts mit einem vorher festgelegten Grenzwert $(\delta)$,
- in Abhängigkeit von diesem Vergleich (106), Auslösen einer Schätzung (108) eines neuen Referenzwerts $(\hat{\lambda})$ des für die Referenzachse repräsentativen Vektors.

2. Nachweisverfahren nach Anspruch 1, wobei die Referenzachse von dem Rechner (22) über ihren repräsentativen Vektor mit Hilfe der von dem Bewegungssensor (18) bereitgestellten Messungen geschätzt wird.

3. Nachweisverfahren nach Anspruch 1 oder 2, aufweisend ferner einen von dem Rechner (22) ausgeführten Schritt (110) des Ungültigmachens von mindestens einem Teil der Sequenz von Zuständen, der der Beobachtungssequenz (O) entspricht, in Abhängigkeit von dem Wahrscheinlichkeitswert.

4. Nachweisverfahren nach einem der Ansprüche 1 bis 3, wobei die Beobachtungssequenz (O) von der Sequenz von Messungen (M) folgendermaßen abgeleitet (102) wird:

- Berechnen einer ersten Komponente (O(1)) aus einem Tiefpass-Filtern mit einer vorher festgelegten Kappungsfrequenz jeder Komponente der Sequenz von Messungen, gefolgt von einer Projektion der resultierenden Komponenten auf den Referenzwert $\hat{\lambda}$,
- Berechnen mindestens einer zweiten Komponente (O(2)) aus einem Bandpass-Filtern mit vorher festgelegten Kappungsfrequenzen jeder Komponente der Sequenz von Messungen.

5. Nachweisverfahren nach einem der Ansprüche 1 bis 4, wobei das statistische Modell (HMM) ein einziges Markov-Modell mit verborgenen Zuständen ist, von dem jeder verborgene Zustand einem der möglichen vorher festgelegten Zustände (K1, K2, K3, K4, K5) entspricht, in denen sich das physikalische System (40) befinden kann, wobei die bedingte Wahrscheinlichkeitsverteilung der Beobachtung jedes verborgenen Zustands von einer Normalverteilungssumme modelliert wird.

6. Nachweisverfahren nach einem der Ansprüche 1 bis 5, wobei die von den Kalibrierungsmitteln (26, 32) ausgeführte Kalibrierung aufweist:

- das Berechnen (106) des Wahrscheinlichkeitswerts V der Beobachtungssequenz (O) vor dem Hintergrund

$$V = \frac{1}{N} \log(p(O_{1:N})$$

des statistischen Modells (HMM) in Form von:                                , wobei N die Anzahl von Beobachtungsmustern in der Beobachtungssequenz (O) ist, wobei $O_{1:N}$ die N aufeinanderfolgenden Muster bezeichnet und $p(O_{1:N})$ ihre Wahrscheinlichkeit,
- einen Vergleich (106) dieses Wahrscheinlichkeitswerts mit einem vorher festgelegten Grenzwert $(\delta)$, wobei dieser Grenzwert aus der Zufallsauswahl einer Vielzahl theoretischer Beobachtungssequenzen auf der Basis des statistischen Modells (HMM) definiert ist, und
- das Auslösen (106) der Schätzung (108) eines neuen Referenzwerts $(\hat{\lambda})$ des für die Referenzachse repräsentativen Vektors nur dann, wenn der Wahrscheinlichkeitswert kleiner als der vorher festgelegte Grenzwert $(\delta)$ ist.

7. Nachweisverfahren nach einem der Ansprüche 1 bis 6, wobei:

- mindestens eine Komponente der Beobachtungssequenz (O) von dem Rechner (22) unabhängig von dem Referenzwert $(\hat{\lambda})$ des für die Referenzachse repräsentativen Vektors berechnet wird,

- mindestens ein Zustand des physischen Systems (40), der einer Sequenz von Messungen entspricht, von einem Aktivitätsnachweismodul (30) auf der Basis dieser Komponente der Beobachtungssequenz (O) nachgewiesen wird, die von dem Referenzwert $(\hat{\lambda})$ unabhängig ist,

- der neue Referenzwert $(\hat{\lambda})$ von dem Rechner (22) aus der Sequenz von Messungen geschätzt wird, die diesem auf der Basis der Komponente der Beobachtungssequenz (O), die von dem Referenzwert $(\hat{\lambda})$ unabhängig ist, nachweisbaren Zustand entspricht.

8. Nachweisverfahren nach Anspruch 7, wobei die Schätzung (108) eines neuen Referenzwerts $(\hat{\lambda})$ des für die Referenzachse repräsentativen Vektors die Schritte aufweist des:

- Nachweisens (200) und Rückgewinnens (202), auf der Basis der mindestens einen Beobachtungssequenzkomponente, die von dem Referenzwert $(\hat{\lambda})$ unabhängig ist, einer Sequenz von Messungen, die einem vorher festgelegten Zustand entspricht, wobei der für die Referenzachse repräsentative Vektor, unter Berücksichtigung des statistischen Modells, im Mittel den Referenzwert haben muss,

- Berechnens (204, 206) der Mittelwerte der Komponenten dieses repräsentativen Vektors in dieser Sequenz von Messungen, die dem vorher festgelegten Zustand entspricht, durch Tiefpass-Filtern (204) mit einer vorher festgelegten Kappungsfrequenz jeder Komponente dieser Sequenz von Messungen, dann Berechnen (206) der Mittel der gefilterten Komponenten, und

- Zuweisens dieser Mittelwerte an den neuen Referenzwert.

9. Anwendung eines Nachweisverfahrens nach einem der Ansprüche 1 bis 7 auf die Beobachtung, mit Hilfe eines dreidimensionalen Beschleunigungsmessers (18), einer Person (40), die sich in jedem Moment in einem der folgenden vorher festgelegten Zustände befinden kann: einem Zustand (K1) "sitzende Position", einem Zustand (K2) "Übergang sitzend/stehend", einem Zustand (K3) "statische stehende Position", einem Zustand (K4) "Gehen" und einem Zustand (K5) "Übergang stehend/sitzend"; wobei der Referenzwert $(\hat{\lambda})$ der Wert ist, den im Bezugssystem der Messachsen des dreidimensionalen Beschleunigungsmessers (18) die Komponenten eines vertikalen und nach unten gerichteten Einzelvektors annehmen, wenn die beobachtete Person (40) den dreidimensionalen Beschleunigungsmesser trägt und im Zustand "statische stehende Position" oder "Gehen" ist.

10. Vorrichtung (10) zum Nachweis der Aktivität (A) eines physischen Systems (40), aufweisend:

- einen Bewegungssensor (18), der bestimmt ist, von dem physischen System (40) für die Bereitstellung einer Sequenz von Messungen (M) getragen zu werden,

- Speichermittel (20) eines statistischen Modells (HMM), das mögliche Werte von Komponenten einer Beobachtungssequenz (O) einer Vielzahl möglicher vorher festgelegter Zustände (K1, K2, K3, K4, K5) zuordnet, in denen sich das physische System (40) befinden kann,

- einen Rechner (22), der mit dem Bewegungssensor (18) und den Speichermitteln (20) verbunden ist, der programmiert ist, um eine Beobachtungssequenz (O) aus der Sequenz von Messungen (M) zu berechnen und um mit Hilfe des statistischen Modells (HMM) die Aktivität (A) des physischen Systems (40) in Form einer Sequenz von Zuständen zu bestimmen, die der Beobachtungssequenz entspricht,

**dadurch gekennzeichnet, dass** der Rechner (22) programmiert (26, 28) ist, um mindestens eine Komponente (O(1)) der Beobachtungssequenz (O) aus einem Referenzwert $(\hat{\lambda})$ eines für eine Referenzachse repräsentativen Vektors zu berechnen, und dadurch, dass die Vorrichtung (10) Kalibriermittel (26, 32) aufweist, die ausgebildet sind, um:

- einen Wahrscheinlichkeitswert der Beobachtungssequenz (O) vor dem Hintergrund des statistischen Modells (HMM) zu berechnen,

- diesen Wahrscheinlichkeitswert mit einem vorher festgelegten Grenzwert $(\delta)$ zu vergleichen,

- in Abhängigkeit von diesem Vergleich eine Schätzung eines neuen Referenzwerts $(\hat{\lambda})$ des für die Referenzachse repräsentativen Vektors auszulösen.

11. Nachweisvorrichtung (10) nach Anspruch 9, wobei der Bewegungssensor (18) ein Beschleunigungsmesser mit drei Messachsen ist, wobei der Rechner programmiert ist, um die Referenzachse, welche die Achse des Erdgravitationsfeldes ist, mit Hilfe der von dem Bewegungssensor bereitgestellten Messungen zu schätzen, wobei der Referenzwert $(\hat{\lambda})$ eines für diese Achse repräsentativen Vektors aus einer Schätzung der Koordinaten einer Beschleunigung, die dem Erdgravitationsfeld im Bezugssystem der Messachsen des Beschleunigungsmessers geschuldet ist, berechnet wird, wenn das physische System (40) in einen vorher festgelegten Zustand ist.

12. System zum Nachweis der Aktivität eines physischen Systems (40), aufweisend:

- einen Gürtel (44), der bestimmt ist, am Torso oder an der Taille einer Person getragen zu werden, und
- ein Gehäuse (42), das an dem Gürtel (44) befestigt ist und eine Nachweisvorrichtung nach Anspruch 9 oder 10 aufweist.

13. Rechnerprogramm, das von einem Kommunikationsnetz herunterladbar und/oder auf einem rechnerlesbaren Träger gespeichert und/oder von einem Prozessor ausführbar ist, **dadurch gekennzeichnet, dass** es Befehle für die Ausführung der Schritte eines Nachweisverfahrens nach einem der Ansprüche 1 bis 8 umfasst, wenn das Programm auf einem Rechner ausgeführt wird.

**Claims**

1. A method for detecting activity (A) of a physical system (40) carrying a motion sensor (18), comprising the following steps executed by a calculator (22):

- extracting (100) a sequence (M) of measurements provided by the motion sensor (18),
- deducing (102) therefrom an observation sequence (O) calculated using the sequence of measurements (M),
- using a statistical model (HMM) of components of the observation sequence (O) in view of a plurality of possible predetermined states (K1, K2, K3, K4, K5) of the physical system (40), determining (104) the activity (A) of the physical system in the form of a sequence of states corresponding to the observation sequence (O),

**characterized in that** at least one component of the observation sequence (O) is calculated on the basis of a reference value $(\hat{\lambda})$ of a vector representing a reference axis, and **in that** the method comprises the following calibration steps executed by calibration means (26, 32):

- calculating (106) a likelihood value for the observation sequence (O) in view of said statistical model (HMM),
- comparing (106) said likelihood value with a predetermined threshold $(\delta)$,
- on the basis of this comparison (106), activating an estimation (108) of a new reference value $(\hat{\lambda})$ for the vector representing the reference axis.

2. The detection method as claimed in claim 1, wherein the reference axis is estimated by the calculator (22), via its representative vector, by means of measurements provided by the motion sensor (18).

3. The detection method as claimed in claim 1 or 2, further comprising a step (110) executed by the calculator (22), of invalidating at least some of the sequence of states corresponding to the observation sequence (O), on the basis of the likelihood value.

4. The detection method as claimed in any one of claims 1 to 3, wherein the observation sequence (O) is deduced (102) from the measurement sequence (M) in the following way:

- calculation of a first component (O(1)), on the basis of a low-pass filtering with a predetermined cutoff frequency of each component of the measurement sequence, then a projection of the resulting components onto the reference value $\hat{\lambda}$,
- calculation of at least one second component (O(2)), on the basis of a band-pass filtering with predetermined cutoff frequencies of each component of the measurement sequence.

5. The detection method as claimed in any one of claims 1 to 4, wherein the statistical model (HMM) is a single Markov model with hidden states, each hidden state of which corresponds to one of the possible predetermined states (K1, K2, K3, K4, K5) of the physical system (40), the law of conditional probability of the observation of each hidden state being modeled by a sum of normal laws.

6. The detection method as claimed in any one of claims 1 to 5, wherein the calibration executed by the calibration means (26, 32) comprises:

- the calculation (106) of the value V of the likelihood of the observation sequence (O) in view of said statistical

$$V = \frac{1}{N}\log\big(p\big(O_{1:N}\big)\big),$$

model (HMM) in the form: where N is the number of observation samples in the observation sequence (O), $O_{1:N}$ designating the N successive samples and $p(O_{1:N})$ the probability thereof,

- a comparison (106) of this likelihood value with a predetermined threshold ($\delta$), this threshold being defined on the basis of the random drawing of a plurality of theoretical observation sequences based on said statistical model (HMM), and

- the activation (106) of the estimation (108) of a new reference value ($\hat{\lambda}$) for the vector representing the reference axis only if the likelihood value is lower than the predetermined threshold ($\delta$).

7. The detection method as claimed in any one of claims 1 to 6, wherein:

- at least one component of the observation sequence (O) is calculated by the calculator (22) independently of the reference value ($\hat{\lambda}$) of the vector representing the reference axis,
- at least one state of the physical system (40) corresponding to a measurement sequence is detected by an activity detection module (30), on the basis of this observation sequence (O) component independent of the reference value ($\hat{\lambda}$),
- the new reference value ($\hat{\lambda}$) is estimated by the calculator (22) on the basis of the measurement sequence corresponding to this state detectable on the basis of the observation sequence (O) component independent of the reference value ($\hat{\lambda}$).

8. The detection method as claimed in claim 7, wherein the estimation (108) of a new reference value ($\hat{\lambda}$) of the vector representing the reference axis comprises steps consisting of:

- detecting (200) and recovering (202), on the basis of said at least one observation sequence component independent of the reference value ($\hat{\lambda}$), a measurement sequence corresponding to a predetermined state wherein the vector representing the reference axis must have, on average, the reference value in consideration of said statistical model,
- calculating (204, 206) the average values of the components of said representative vector in this measurement sequence corresponding to the predetermined state, by low-pass filtering (204) with a predetermined cutoff frequency of each component of this measurement sequence, then calculating (206) averages of the filtered components, and
- assigning these average values to the new reference value.

9. An application of the detection method as claimed in any one of claims 1 to 7, to the observation, by means of a three-dimensional accelerometer (18), of a person (40) capable of being, at each instant, in one of the following predetermined states: a "sitting position" state (K1), a "sitting/standing transition" state (K2), a "static standing position" state (K3), a "walking" state (K4) and a "standing/sitting transition" state (K5); the reference value ($\hat{\lambda}$) being the value, in the frame of the measurement axes of the three-dimensional accelerometer (18), of the components of a vertical downward-oriented unit vector when the person being observed (40) is wearing the three-dimensional accelerometer and is in the "static standing position" or "walking" state.

10. A device (10) for detecting the activity (A) of a physical system (40) comprising:

- a motion sensor (18) intended to be carried by the physical system (40) in order to provide a sequence of measurements (M),
- means (20) for storing a statistical model (HMM) associating possible values of components of an observation sequence (O) with a plurality of possible predetermined states (K1, K2, K3, K4, K5) of the physical system (40),
- a calculator (22), connected to the motion sensor (18) and to the storage means (20), programmed to calculate an observation sequence (O) on the basis of the measurement sequence (M) and to determine, using the statistical model (HMM), the activity (A) of the physical system (40) in the form of a sequence of states corresponding to the observation sequence,

**characterized in that** the calculator (22) is programmed (26, 28) to calculate at least one component (O(1)) of the observation sequence (O) on the basis of a reference value ($\hat{\lambda}$) of a vector representing a reference axis, and **in that** the device (10) comprises calibration means (26, 32) designed to:

- calculate a likelihood value for the observation sequence (O) in view of said statistical model (HMM),
- compare this likelihood value with a predetermined threshold ($\delta$),
- activate an estimation of a new reference value ($\hat{\lambda}$) for the vector representing the reference axis on the basis of this comparison.

11. The detection device (10) as claimed in claim 9, wherein the motion sensor (18) is an accelerometer with three measurement axes, the calculator being programmed to estimate the reference axis by means of the measurements provided by the motion sensor being the axis of the earth's gravitational field, the reference value ($\hat{\lambda}$) of a vector representing this axis being calculated on the basis of an estimation of the coordinates of an acceleration due to the earth's gravitational field in the frame of reference of the measurement axes of the accelerometer when the physical system (40) is in a predetermined state.

12. A system for detecting the activity of a physical system (40), comprising:

   - a belt (44) intended to be worn on the torso or the waist of a person, and
   - a housing (42) secured to the belt (44) and comprising the detection device as claimed in claim 9 or 10.

13. A computer program that can be downloaded from a communication network and/or recorded on a computer-readable medium and/or run by a processor, **characterized in that** it comprises instructions for carrying out the steps of the detection method as claimed in any one of claims 1 to 8, when said program is run on a computer.

## Figure 1

## Figure 2

## Figure 3

$a_{3,3}$

$a_{1,1}$   $a_{2,2}$   $a_{2,3}$   K3   $a_{3,5}$   $a_{5,5}$

K1   $a_{1,2}$   K2   $a_{3,4}$   $a_{4,3}$   K5

HMM   $a_{2,4}$   K4   $a_{4,5}$

$a_{4,4}$   $a_{5,1}$

## Figure 4

100   M   102   O   104   A   106

$\hat{\lambda}$

110   108

# Figure 5

# Figure 6

# Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010122174 A1

**Littérature non-brevet citée dans la description**

- A graph based algorithm for postures estimation based on accelerometers data. *Proceedings of 2010 annual international conférence of the IEEE EMBC,* 2010, 2778-2781